# EUROPEAN PATENT APPLICATION

(11) **EP 4 636 097 A1**
(43) Date of publication of application: **22.10.2025**
(21) Application number: 24382407.5
(22) Date of filing: 17.04.2024
(51) Int. Cl.: C12Q 1/6886

(54) **BIOMARKERS FOR PREDICTING THE RESPONSE OF A PATIENT SUFFERING FROM SOFT TISSUE SARCOMA TO TREATMENT WITH A COMBINED THERAPY COMPRISING A PD-1/PD-L1 INHIBITOR AND A TYROSINE KINASE INHIBITOR**

(71) Applicant: Fundación Instituto de Investigación Sanitaria Fundación Jiménez Díaz, 28040 Madrid (ES)
(72) Inventor: MARTÍN-BROTO, Javier, 28040 Madrid (ES); MOURA, David da Silva, 28040 Madrid (ES)
(74) Representative: Hoffmann Eitle

(57) **Abstract**

**INHIBITOR.** The present invention refers to methods for predicting the response of a patient suffering from soft tissue sarcoma to treatment with a combined therapy comprising a PD-1/PD-L1 inhibitor and a tyrosine kinase inhibitor.

## Description

### FIELD OF THE INVENTION

The present invention refers to the medical field. Particularly, the present invention refers to methods for predicting the response of a patient suffering from soft tissue sarcoma to treatment with a combined therapy comprising a PD-1/PD-L1 inhibitor and a tyrosine kinase inhibitor.

### STATE OF THE ART

Sarcomas encompass over fifty types of bone and soft tissue tumours. They are uncommon and typically present a poor outcome. Sarcomas can occur at any age and are not restricted to a specific body location. As in other cancer types, the starting point is the histopathological characterization of the primary tumour, afterwards, several methods are used to predict cancer progression measured as Progression-Free survival (PFS) and Overall Survival.

Over the last few years several gene signatures have been proposed seeking biomarkers with predictive power on drug therapies and complex genetic backgrounds. In line with those studies, the inventors conducted a Phase-I/II correlative clinical trial in sixty-four patients from a variety of soft-tissue sarcoma subtypes treated with a combined therapy of nivolumab and sunitinib. A combination of both drugs has been proved to be an active scheme to treat patients with advanced soft tissue sarcomas. Nivolumab is a monoclonal antibody that binds to PD-1 receptor unblocking the PD-1 mediated inhibition of the immune response. Besides, sunitinib is an antiangiogenic drug that inhibits different tyrosine kinase receptors like platelet-derived growth factor receptor (PDGF-R) and vascular endothelial growth factor receptor (VEGF-R).

While the combination of the PD-1/PD-L1 inhibitor nivolumab and the tyrosine kinase inhibitor sunitinib has been found to be effective for the treatment of soft tissue sarcoma, there is still a need for the development of strategies suitable for predicting the response of a patient suffering from soft tissue sarcoma to said treatment, which would provide clinicians with a reliable tool for selecting those patients who would benefit from the treatment and differentiating them from non-responder patients who would receive an alternative treatment or surveillance.

The present invention is focused on solving this problem and a method for predicting the response of a patient suffering from soft tissue sarcoma to treatment with a combined therapy comprising a PD-1/PD-L1 inhibitor and a tyrosine kinase inhibitor are provided herein.

### DESCRIPTION OF THE INVENTION

### Brief description of the invention

The present invention refers to an *in vitro* method for predicting the response of a patient suffering from soft tissue sarcoma to treatment with a combined therapy comprising a PD-1/PD-L1 inhibitor and a tyrosine kinase inhibitor, for classifying a patient suffering from soft tissue sarcoma into responder or non-responder patient to treatment with a combined therapy comprising a PD-1/PD-L1 inhibitor and a tyrosine kinase inhibitor, for monitoring patients suffering from soft tissue sarcoma to assess whether they are responding to treatment with a combined therapy comprising a PD-1/PD-L1 inhibitor and a tyrosine kinase inhibitor, or for the identification of biomarker signatures suitable for predicting the response of a patient suffering from soft tissue sarcoma to treatment with a combined therapy comprising a PD-1/PD-L1 inhibitor and a tyrosine kinase inhibitor.

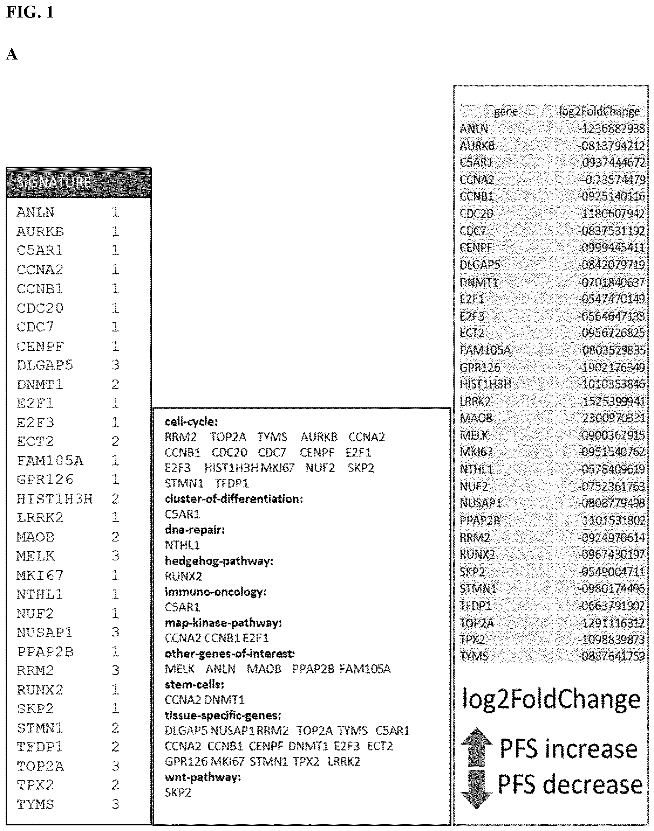

Particularly, by comparing transcriptomic data from tumour biopsies obtained from soft tissue sarcoma patients before treatment with a PD-1/PD-L1 inhibitor and a tyrosine kinase inhibitor, particularly nivolumab and sunitinib, the inventors of the present invention identified 32 genes that were differentially expressed in patients with a progression free survival (PFS) ≥ 6 months (n=38) versus patients with PFS < 6 months (n=26), which are: *ANLN, AURKB, C5AR1, CCNA2, CCNB1, CDC20, CDC7, CENPF, DLGAP5, DNMT1, E2F1, E2F3, ECT2, FAM105A, GPR126, HIST1H3H, LRRK2, MAOB, MELK, MKI67, NTHL1, NUF2, NUSAP1, PPAP2B, RRM2, RUNX2, SKP2, STMN1, TFDP1, TOP2A, TPX2* and *TYMS* **(****Figure 1a****).**

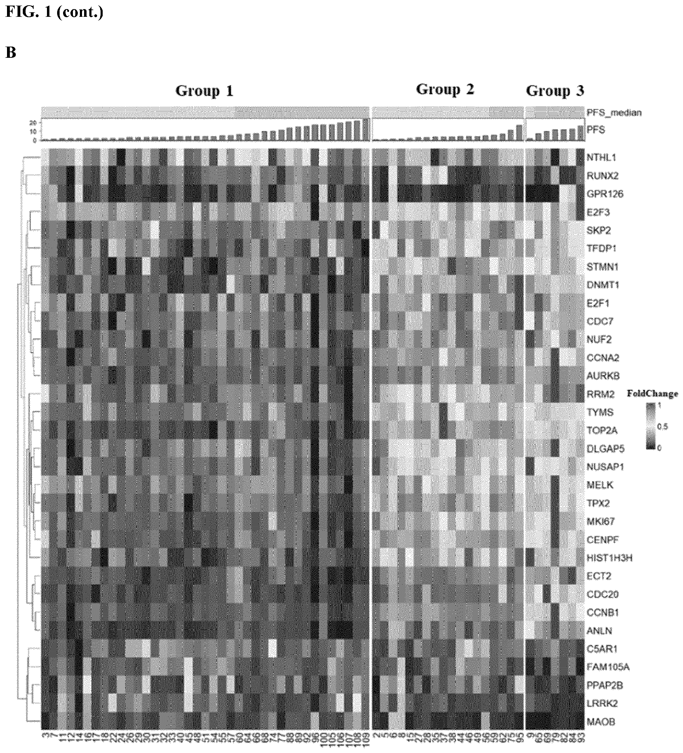

These 32 genes successfully clustered the study population in three separated risk groups that significantly differed in their PFS after treatment with nivolumab and sunitinib **(****Figure 1b****-d).**

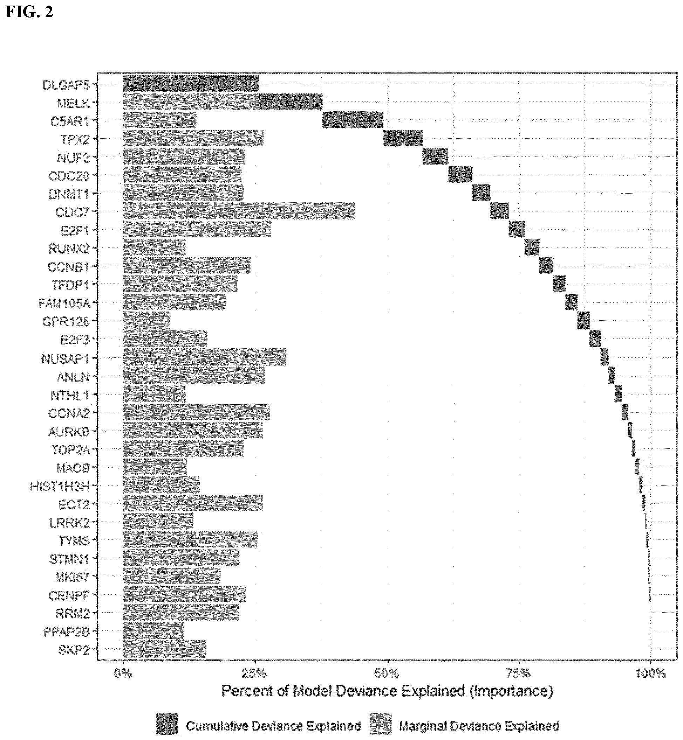

The marginal and accumulative importance of each gene on the PFS gene signature was represented in Generalized Linear Model (GLM) variable importance plots, showing than the combination of only 9 genes *(DLGAP5, MELK, C5AR1, TPX2, NUF2, CDC20, DNMT1, CDC7* and *E2F1)* explained more than 75% of the PFS variance **(****Figure 2****).**

Thus, the inventors have identified 32 genes that can be used (either as individual biomarkers or as signatures comprising between two and 32 genes) for predicting the response of a patient suffering from soft tissue sarcoma to treatment with a combined therapy comprising a PD-1/PD-L1 inhibitor and a tyrosine kinase inhibitor, for instance nivolumab and sunitinib.

Of note, a combination of 9 genes (*DLGAP5, MELK, CSAR1, TPX2, NUF2, CDC20, DNMT1, CDC7* and *E2F1)* was particularly selected from these 32 genes that explain 75% of the PFS variance.

So, these experiments support the use of the 32 genes (*ANLN, AURKB, C5AR1, CCNA2, CCNB1, CDC20, CDC7, CENPF, DLGAP5, DNMT1, E2F1, E2F3, ECT2, FAM105A, GPR126, HIST1H3H, LRRK2, MAOB, MELK, MKI67, NTHL1, NUF2, NUSAP1, PPAP2B, RRM2, RUNX2, SKP2, STMN1, TFDP1, TOP2A, TPX2* and *TYMS*)*,* either as individual biomarkers or as signatures comprising between two and 32 genes, and more specifically the use of the 9 genes (*DLGAP5, MELK, C5AR1, TPX2, NUF2, CDC20, DNMT1, CDC7* and *E2F1)* either as individual biomarkers or as signatures comprising between two and 9 genes, for predicting the response of a patient suffering from soft tissue sarcoma to treatment with a combined therapy comprising a PD-1/PD-L1 inhibitor, such as nivolumab, and a tyrosine kinase inhibitor, such as sunitinib.

In view of the above-mentioned results, a first aspect of the invention refers to an *in vitro* method (hereinafter referred to as the first *in vitro* method of the invention) for the identification of biomarker signatures suitable for predicting the response of a patient suffering from soft tissue sarcoma to treatment with a combined therapy comprising a PD-1/PD-L1 inhibitor and a tyrosine kinase inhibitor, or for classifying a patient suffering from soft tissue sarcoma into responder or non-responder patient to a treatment with a combined therapy comprising a PD-1/PD-L1 inhibitor and a tyrosine kinase inhibitor, or for monitoring patients suffering from soft tissue sarcoma to assess whether they are responding to a treatment with a combined therapy comprising a PD-1/PD-L1 inhibitor and a tyrosine kinase inhibitor, or for deciding or recommending whether to treat a patient suffering from soft tissue sarcoma treatment with a combined therapy comprising a PD-1/PD-L1 inhibitor and a tyrosine kinase inhibitor which comprises:
a) assessing the level of expression of at least two genes selected from the list consisting of DLGAP5, ANLN, AURKB, C5AR1, CCNA2, CCNB 1, CDC20, CDC7, CENPF, DNMT1, E2F1, E2F3, ECT2, FAM105A, GPR126, HIST1H3H, LRRK2, MAOB, MELK, MKI67, NTHL1, NUF2, NUSAP1, PPAP2B, RRM2, RUNX2, SKP2, STMN1, TFDP1, TOP2A, TPX2 and/or TYMS, or the enrichment of the signature comprising said genes, in a biological sample obtained from a patient suffering from soft tissue sarcoma who is a responder patient to treatment with a combined therapy comprising a PD-1/PD-L1 inhibitor and a tyrosine kinase inhibitor,
b) assessing the level of expression of the at least two genes selected from the list consisting of DLGAP5, ANLN, AURKB, C5AR1, CCNA2, CCNB1, CDC20, CDC7, CENPF, DNMT1, E2F1, E2F3, ECT2, FAM105A, GPR126, HIST1H3H, LRRK2, MAOB, MELK, MKI67, NTHL1, NUF2, NUSAP1, PPAP2B, RRM2, RUNX2, SKP2, STMN1, TFDP1, TOP2A, TPX2 and/or TYMS, or the enrichment of the signature comprising said genes, in a biological sample obtained from a patient suffering from soft tissue sarcoma who is a non-responder patient to treatment with a combined therapy comprising a PD-1/PD-L1 inhibitor and a tyrosine kinase inhibitor,
c) wherein the identification of a deviation in the expression level of the at least two genes selected from selected from the list consisting of DLGAP5, ANLN, AURKB, C5AR1, CCNA2, CCNB1, CDC20, CDC7, CENPF, DNMT1, E2F1, E2F3, ECT2, FAM105A, GPR126, HIST1H3H, LRRK2, MAOB, MELK, MKI67, NTHL1, NUF2, NUSAP1, PPAP2B, RRM2, RUNX2, SKP2, STMN1, TFDP1, TOP2A, TPX2 and/or TYMS, or in the enrichment of the signature comprising said genes, across responder and non-responder patients, is an indication that the signature is suitable for predicting the response of a patient suffering from soft tissue sarcoma to treatment with a combined therapy comprising a PD-1/PD-L1 inhibitor and a tyrosine kinase inhibitor, or for classifying a patient suffering from soft tissue sarcoma into responder or non-responder patients to a treatment with a combined therapy comprising a PD-1/PD-L1 inhibitor and a tyrosine kinase inhibitor, or for monitoring patients suffering from soft tissue sarcoma to assess whether they are responding to a treatment with a combined therapy comprising a PD-1/PD-L1 inhibitor and a tyrosine kinase inhibitor, or for deciding or recommending whether to treat a patient suffering from soft tissue sarcoma treatment with a combined therapy comprising a PD-1/PD-L1 inhibitor and a tyrosine kinase inhibitor.

In a preferred embodiment the first *in vitro* method of the invention comprises:
a) assessing the level of expression of at least two genes selected from the list consisting of DLGAP5, CDC7, CDC20, C5AR1, DNMT1, E2F1, MELK, NUF2 and/or TPX2, or the enrichment of the signature comprising said genes, in a biological sample obtained from a patient suffering from soft tissue sarcoma who is a responder patient to treatment with a combined therapy comprising a PD-1/PD-L1 inhibitor and a tyrosine kinase inhibitor,
b) assessing the level of expression of the at least two genes selected from the list consisting of DLGAP5, CDC7, CDC20, C5AR1, DNMT1, E2F1, MELK, NUF2 and/or TPX2, or the enrichment of the signature comprising said genes, in a biological sample obtained from a patient suffering from soft tissue sarcoma who is a non-responder patient to treatment with a combined therapy comprising a PD-1/PD-L1 inhibitor and a tyrosine kinase inhibitor,
c) wherein the identification of a deviation in the expression level of the at least two genes selected from selected from the list consisting of DLGAP5, CDC7, CDC20, C5AR1, DNMT1, E2F1, MELK, NUF2 and/or TPX2, or the enrichment of the signature comprising said genes, across responder and non-responder patients, is an indication that the signature is suitable for predicting the response of a patient suffering from soft tissue sarcoma to treatment with a combined therapy comprising a PD-1/PD-L1 inhibitor and a tyrosine kinase inhibitor, or for classifying a patient suffering from soft tissue sarcoma into responder or non-responder patients to a treatment with a combined therapy comprising a PD-1/PD-L1 inhibitor and a tyrosine kinase inhibitor, or for monitoring patients suffering from soft tissue sarcoma to assess whether they are responding to a treatment with a combined therapy comprising a PD-1/PD-L1 inhibitor and a tyrosine kinase inhibitor, or for deciding or recommending whether to treat a patient suffering from soft tissue sarcoma treatment with a combined therapy comprising a PD-1/PD-L1 inhibitor and a tyrosine kinase inhibitor.

In a preferred embodiment, the soft tissue sarcoma is selected from the list consisting of clear cell sarcoma, synovial sarcoma, undifferentiated pleomorphic sarcoma, alveolar soft-part sarcoma, angiosarcoma, epithelioid sarcoma, solitary fibrous tumour, extraskeletal myxoid chondrosarcoma and/or epithelioid hemangioendothelioma. Preferably, the soft tissue sarcoma is selected from the list consisting of clear cell sarcoma, synovial sarcoma and/or undifferentiated pleomorphic sarcoma.

In a preferred embodiment, the PD-1/PD-L1 inhibitor is selected from: a) PD-1 inhibitor selected from: nivolumab, pembrolizumab, cemiplimab, dostarlimab, retifanlimab and/or toripalimab, and/or b) a PD-L1 inhibitor selected from: atezolizumab, avelumab and/or durvalumab. Preferably, the PD-1/PD-L1 inhibitor is a PD-1 inhibitor selected from: atezolizumab, avelumab and/or durvalumab. More preferably, the PD-1/PD-L1 inhibitor is nivolumab.

In a preferred embodiment, the tyrosine kinase inhibitor is selected from: sunitinib, pazopanib, cediranib, axitinib, anlotinib, apatinib, sorafenib and/or regorafenib. Preferably, the tyrosine kinase inhibitor is sunitinib.

In a preferred embodiment, the PD-1/PD-L1 inhibitor is nivolumab and/or the tyrosine kinase inhibitor is sunitinib.

In a preferred embodiment, a responder is a patient that shows an increased progression free survival response compared to a pre-established value.

In a preferred embodiment, a responder is a patient that shows a decreased progression free survival response compared to a pre-established value.

In a preferred embodiment, the biological sample is a soft tissue sarcoma tissue biopsy.

A second aspect of the invention refers to an *in vitro* method (hereinafter referred to as the second *in vitro* method of the invention) for predicting the response of a patient suffering from soft tissue sarcoma to treatment with a combined therapy comprising a PD-1/PD-L1 inhibitor and a tyrosine kinase inhibitor, or for classifying a patient suffering from soft tissue sarcoma into responder or non-responder patients to a treatment with a combined therapy comprising a PD-1/PD-L1 inhibitor and a tyrosine kinase inhibitor, or for monitoring patients suffering from soft tissue sarcoma to assess whether they are responding to treatment with a combined therapy comprising a PD-1/PD-L1 inhibitor and a tyrosine kinase inhibitor, which comprises:
a) assessing the level of expression of a gene, or any combination comprising from 2 to 32 genes, selected from the list consisting of DLGAP5, ANLN, AURKB, C5AR1, CCNA2, CCNB1, CDC20, CDC7, CENPF, DNMT1, E2F1, E2F3, ECT2, GPR126, HIST1H3H, LRRK2, MAOB, MELK, MKI67, NTHL1, NUF2, NUSAP1, RRM2, RUNX2, SKP2, STMN1, TFDP1, TOP2A, TPX2 and/or TYMS in a biological sample obtained from the patient,
b) wherein the identification of a decreased expression level of the genes DLGAP5, ANLN, AURKB, CCNA2, CCNB1, CDC20, CDC7, CENPF, DNMT1, E2F1, E2F3, ECT2, FAM105A, GPR126, HIST1H3H, MELK, MKI67, NTHL1, NUF2, NUSAP1, PPAP2B, RRM2, RUNX2, SKP2, STMN1, TFDP1, TOP2A, TPX2 and/or TYMS and an increased expression level of genes C5AR1, FAM105A, LRRK2, MAOB and/or PPAP2B, as compared with a pre-established threshold value, is an indication that the patient will respond to treatment with a combined therapy comprising a PD-1/PD-L1 inhibitor and a tyrosine kinase inhibitor, or that the patient is responding to treatment with a combined therapy comprising a PD-1/PD-L1 inhibitor and a tyrosine kinase inhibitor,
c) wherein the identification of an increased expression level of the genes DLGAP5, ANLN, AURKB, CCNA2, CCNB1, CDC20, CDC7, CENPF, DNMT1, E2F1, E2F3, ECT2, FAM105A, GPR126, HIST1H3H, MELK, MKI67, NTHL1, NUF2, NUSAP1, PPAP2B, RRM2, RUNX2, SKP2, STMN1, TFDP1, TOP2A, TPX2 and/or TYMS and a decreased expression level of genes C5AR1, FAM105A, LRRK2, MAOB and/or PPAP2B, as compared with a pre-established threshold value, is an indication that the patient will not respond to treatment with a combined therapy comprising a PD-1/PD-L1 inhibitor and a tyrosine kinase inhibitor, or that the patient is not responding to treatment with a combined therapy comprising a PD-1/PD-L1 inhibitor and a tyrosine kinase inhibitor,
d) wherein the pre-established threshold value is determined in patients that do not respond to treatment with a combined therapy comprising a PD-1/PD-L1 inhibitor and a tyrosine kinase inhibitor, or by comparing patients that respond to treatment with a combined therapy comprising a PD-1/PD-L1 inhibitor and a tyrosine kinase inhibitor with patients that do not respond to treatment with a combined therapy comprising a PD-1/PD-L1 inhibitor and a tyrosine kinase inhibitor.

In a preferred embodiment the second *in vitro* method of the invention comprises:
a) assessing the level of expression of a combination of at least two genes selected from the list consisting of DLGAP5, CDC7, CDC20, C5AR1, DNMT1, E2F1, MELK, NUF2 and/or TPX2 in a biological sample obtained from the patient,
b) wherein the identification of a decreased expression level of the genes DLGAP5, CDC7, CDC20, DNMT1, E2F1, MELK, NUF2 and/or TPX2 and an increased expression level of gene C5AR1, as compared with a pre-established threshold value, is an indication that the patient will respond to treatment with a combined therapy comprising a PD-1/PD-L1 inhibitor and a tyrosine kinase inhibitor, or that the patient is responding to treatment with a combined therapy comprising a PD-1/PD-L1 inhibitor and a tyrosine kinase inhibitor,
c) wherein the identification of an increased expression level of the genes DLGAP5, CDC7, CDC20, DNMT1, E2F1, MELK, NUF2 and/or TPX2 and a decreased expression level of gene C5AR1, as compared with a pre-established threshold value, is an indication that the patient will not respond to treatment with a combined therapy comprising a PD-1/PD-L1 inhibitor and a tyrosine kinase inhibitor, or that the patient is not responding to treatment with a combined therapy comprising a PD-1/PD-L1 inhibitor and a tyrosine kinase inhibitor,
d) wherein the pre-established threshold value is determined in patients that do not respond to treatment with a combined therapy comprising a PD-1/PD-L1 inhibitor and a tyrosine kinase inhibitor, or by comparing patients that respond to treatment with a combined therapy comprising a PD-1/PD-L1 inhibitor and a tyrosine kinase inhibitor with patients that do not respond to treatment with a combined therapy comprising a PD-1/PD-L1 inhibitor and a tyrosine kinase inhibitor.

Preferably, the combination of genes consists of DLGAP5, CDC7, CDC20, C5AR1, DNMT1, E2F1, MELK, NUF2 and TPX2.

More preferably, the combination of genes consists of DLGAP5, CDC7, CDC20, C5AR1, DNMT1, E2F1, MELK, NUF2 and TPX2, the biological sample is a soft tissue sarcoma tissue biopsy, the PD-1/PD-L1 inhibitor is nivolumab and the tyrosine kinase inhibitor is sunitinib.

In a more preferred embodiment, the second *in vitro* method of the invention comprises:
a) assessing the level of expression of the combination of the genes DLGAP5, CDC7, CDC20, C5AR1, DNMT1, E2F1, MELK, NUF2 and TPX2 in a biological sample obtained from the patient,
b) wherein the identification of a decreased expression level of the genes DLGAP5, CDC7, CDC20, DNMT1, E2F1, MELK, NUF2 and TPX2 and an increased expression level of gene C5AR1, as compared with a pre-established threshold value, is an indication that the patient will respond to treatment with a combined therapy comprising a PD-1/PD-L1 inhibitor and a tyrosine kinase inhibitor, or that the patient is responding to treatment with a combined therapy comprising a PD-1/PD-L1 inhibitor and a tyrosine kinase inhibitor,
c) wherein the identification of an increased expression level of the genes DLGAP5, CDC7, CDC20, DNMT1, E2F1, MELK, NUF2 and TPX2 and a decreased expression level of gene C5AR1, as compared with a pre-established threshold value, is an indication that the patient will not respond to treatment with a combined therapy comprising a PD-1/PD-L1 inhibitor and a tyrosine kinase inhibitor, or that the patient is not responding to treatment with a combined therapy comprising a PD-1/PD-L1 inhibitor and a tyrosine kinase inhibitor,
d) wherein the pre-established threshold value is determined in patients that do not respond to treatment with a combined therapy comprising a PD-1/PD-L1 inhibitor and a tyrosine kinase inhibitor, or by comparing patients that respond to treatment with a combined therapy comprising a PD-1/PD-L1 inhibitor and a tyrosine kinase inhibitor with patients that do not respond to treatment with a combined therapy comprising a PD-1/PD-L1 inhibitor and a tyrosine kinase inhibitor.

Preferably, the biological sample is a soft tissue sarcoma tissue biopsy, the PD-1/PD-L1 inhibitor is nivolumab and the tyrosine kinase inhibitor is sunitinib.

The soft tissue sarcomas, PD-1/PD-L1 inhibitors, tyrosine kinase inhibitors and biological samples are the same as described for the first aspect. Thus, all the embodiments of the description mentioned in these regards for the first aspect apply herein.

A third aspect of the invention refers to an *in vitro* method (hereinafter referred to as the third method of the invention) for deciding or recommending whether to treat a patient suffering from soft tissue sarcoma treatment with a combined therapy comprising a PD-1/PD-L1 inhibitor and a tyrosine kinase inhibitor, which comprises:
a) assessing the level of expression of a gene, or any combination comprising from 2 to 32 genes, selected from the list consisting of DLGAP5, ANLN, AURKB, C5AR1, CCNA2, CCNB1, CDC20, CDC7, CENPF, DNMT1, E2F1, E2F3, ECT2, GPR126, HIST1H3H, LRRK2, MAOB, MELK, MKI67, NTHL1, NUF2, NUSAP1, RRM2, RUNX2, SKP2, STMN1, TFDP1, TOP2A, TPX2 and/or TYMS in a biological sample obtained from the patient,
b) wherein the identification of a decreased expression level of the genes DLGAP5, ANLN, AURKB, CCNA2, CCNB1, CDC20, CDC7, CENPF, DNMT1, E2F1, E2F3, ECT2, FAM105A, GPR126, HIST1H3H, MELK, MKI67, NTHL1, NUF2, NUSAP1, PPAP2B, RRM2, RUNX2, SKP2, STMN1, TFDP1, TOP2A, TPX2 and/or TYMS and a increased expression level of genes C5AR1, FAM105A, LRRK2, MAOB and/or PPAP2B, as compared with a pre-established threshold value, is an indication that treatment with a combined therapy comprising a PD-1/PD-L1 inhibitor and a tyrosine kinase inhibitor is recommended,
c) wherein the identification of an increased expression level of the genes DLGAP5, ANLN, AURKB, CCNA2, CCNB1, CDC20, CDC7, CENPF, DNMT1, E2F1, E2F3, ECT2, FAM105A, GPR126, HIST1H3H, MELK, MKI67, NTHL1, NUF2, NUSAP1, PPAP2B, RRM2, RUNX2, SKP2, STMN1, TFDP1, TOP2A, TPX2 and/or TYMS and a decreased expression level of genes C5AR1, FAM105A, LRRK2, MAOB and/or PPAP2B, as compared with a pre-established threshold value, is an indication that treatment with a combined therapy comprising a PD-1/PD-L1 inhibitor and a tyrosine kinase inhibitor is not recommended,
d) wherein the pre-established threshold value is determined in patients that do not respond to treatment with a combined therapy comprising a PD-1/PD-L1 inhibitor and a tyrosine kinase inhibitor, or by comparing patients that respond to treatment with a combined therapy comprising a PD-1/PD-L1 inhibitor and a tyrosine kinase inhibitor with patients that do not respond to treatment with a combined therapy comprising a PD-1/PD-L1 inhibitor and a tyrosine kinase inhibitor.

In a preferred embodiment the third *in vitro* method of the invention comprises:
a) assessing the level of expression of a combination of at least two genes selected from the list consisting of DLGAP5, CDC7, CDC20, C5AR1, DNMT1, E2F1, MELK, NUF2 and/or TPX2 in a biological sample obtained from the patient,
b) wherein the identification of a decreased expression level of the genes DLGAP5, CDC7, CDC20, DNMT1, E2F1, MELK, NUF2 and/or TPX2 and an increased expression level of gene C5AR1, as compared with a pre-established threshold value, is an indication that treatment with a combined therapy comprising a PD-1/PD-L1 inhibitor and a tyrosine kinase inhibitor is recommended,
c) wherein the identification of an increased expression level of the genes DLGAP5, CDC7, CDC20, DNMT1, E2F1, MELK, NUF2 and/or TPX2 and a decreased expression level of gene C5AR1, as compared with a pre-established threshold value, is an indication that treatment with a combined therapy comprising a PD-1/PD-L1 inhibitor and a tyrosine kinase inhibitor is not recommended,
d) wherein the pre-established threshold value is determined in patients that do not respond to treatment with a combined therapy comprising a PD-1/PD-L1 inhibitor and a tyrosine kinase inhibitor, or by comparing patients that respond to treatment with a combined therapy comprising a PD-1/PD-L1 inhibitor and a tyrosine kinase inhibitor with patients that do not respond to treatment with a combined therapy comprising a PD-1/PD-L1 inhibitor and a tyrosine kinase inhibitor.

Preferably, the combination of genes consists of DLGAP5, CDC7, CDC20, C5AR1, DNMT1, E2F1, MELK, NUF2 and TPX2.

More preferably, the combination of genes consists of DLGAP5, CDC7, CDC20, C5AR1, DNMT1, E2F1, MELK, NUF2 and TPX2, the biological sample is a soft tissue sarcoma tissue biopsy, the PD-1/PD-L1 inhibitor is nivolumab and the tyrosine kinase inhibitor is sunitinib.

In a more preferred embodiment, the third *in vitro* method of the invention comprises:
a) assessing the level of expression of the combination of the genes DLGAP5, CDC7, CDC20, C5AR1, DNMT1, E2F1, MELK, NUF2 and TPX2 in a biological sample obtained from the patient,
b) wherein the identification of a decreased expression level of the genes DLGAP5, CDC7, CDC20, DNMT1, E2F1, MELK, NUF2 and TPX2 and an increased expression level of gene C5AR1, as compared with a pre-established threshold value, is an indication that treatment with a combined therapy comprising a PD-1/PD-L1 inhibitor and a tyrosine kinase inhibitor is recommended,
c) wherein the identification of an increased expression level of the genes DLGAP5, CDC7, CDC20, DNMT1, E2F1, MELK, NUF2 and TPX2 and a decreased expression level of gene C5AR1, as compared with a pre-established threshold value, is an indication that treatment with a combined therapy comprising a PD-1/PD-L1 inhibitor and a tyrosine kinase inhibitor is not recommended,
d) wherein the pre-established threshold value is determined in patients that do not respond to treatment with a combined therapy comprising a PD-1/PD-L1 inhibitor and a tyrosine kinase inhibitor, or by comparing patients that respond to treatment with a combined therapy comprising a PD-1/PD-L1 inhibitor and a tyrosine kinase inhibitor with patients that do not respond to treatment with a combined therapy comprising a PD-1/PD-L1 inhibitor and a tyrosine kinase inhibitor.

Preferably, the biological sample is a soft tissue sarcoma tissue biopsy, the PD-1/PD-L1 inhibitor is nivolumab and the tyrosine kinase inhibitor is sunitinib.

The soft tissue sarcomas, PD-1/PD-L1 inhibitors, tyrosine kinase inhibitors and biological samples are the same as described for the first aspect. Thus, all the embodiments of the description mentioned in these regards for the first aspect apply herein.

A fourth aspect of the invention refers to the *in vitro* use of the level of expression of a combination of at least two genes selected from the list consisting of DLGAP5, CDC7, CDC20, C5AR1, DNMT1, E2F1, MELK, NUF2 and/or TPX2 for predicting the response of a patient suffering from soft tissue sarcoma to treatment with a combined therapy comprising a PD-1/PD-L1 inhibitor and a tyrosine kinase inhibitor, for classifying a patient suffering from soft tissue sarcoma into responder or non-responder patients to treatment with a combined therapy comprising a PD-1/PD-L1 inhibitor and a tyrosine kinase inhibitor, for monitoring patients suffering from soft tissue sarcoma to assess whether they are responding to treatment with a combined therapy comprising a PD-1/PD-L1 inhibitor and a tyrosine kinase inhibitor, or for the identification of biomarker signatures suitable for predicting the response of a patient suffering from soft tissue sarcoma to treatment with a combined therapy comprising a PD-1/PD-L1 inhibitor and a tyrosine kinase inhibitor.

In a preferred embodiment, the combination of genes consists of DLGAP5, CDC7, CDC20, C5AR1, DNMT1, E2F1, MELK, NUF2 and TPX2.

In a more preferred embodiment, the combination of genes consists of DLGAP5, CDC7, CDC20, C5AR1, DNMT1, E2F1, MELK, NUF2 and TPX2, the biological sample is a soft tissue sarcoma tissue biopsy, the PD-1/PD-L1 inhibitor is nivolumab and the tyrosine kinase inhibitor is sunitinib.

The soft tissue sarcomas, PD-1/PD-L1 inhibitors, tyrosine kinase inhibitors and biological samples are the same as described for the first aspect. Thus, all the embodiments of the description mentioned in these regards for the first aspect apply herein.

A fifth aspect of the invention refers to the *in vitro* use of a kit comprising reagents for the assessment of the level of expression of a combination of at least two genes selected from the list consisting of DLGAP5, CDC7, CDC20, C5AR1, DNMT1, E2F1, MELK, NUF2 and/or TPX2 for predicting the response of a patient suffering from soft tissue sarcoma to treatment with a combined therapy comprising a PD-1/PD-L1 inhibitor and a tyrosine kinase inhibitor, for classifying a patient suffering from soft tissue sarcoma into responder or non-responder patients to treatment with a combined therapy comprising a PD-1/PD-L1 inhibitor and a tyrosine kinase inhibitor, for monitoring patients suffering from soft tissue sarcoma to assess whether they are responding to treatment with a combined therapy comprising a PD-1/PD-L1 inhibitor and a tyrosine kinase inhibitor, or for the identification of biomarker signatures suitable for predicting the response of a patient suffering from soft tissue sarcoma to treatment with a combined therapy comprising a PD-1/PD-L1 inhibitor and a tyrosine kinase inhibitor.

In a preferred embodiment, the combination of genes consists of DLGAP5, CDC7, CDC20, C5AR1, DNMT1, E2F1, MELK, NUF2 and TPX2.

In a more preferred embodiment, the combination of genes consists of DLGAP5, CDC7, CDC20, C5AR1, DNMT1, E2F1, MELK, NUF2 and TPX2, the biological sample is a soft tissue sarcoma tissue biopsy, the PD-1/PD-L1 inhibitor is nivolumab and the tyrosine kinase inhibitor is sunitinib.

The soft tissue sarcomas, PD-1/PD-L1 inhibitors, tyrosine kinase inhibitors and biological samples are the same as described for the first aspect. Thus, all the embodiments of the description mentioned in these regards for the first aspect apply herein.

A sixth aspect of the invention refers to a combination drug product for use in a method of treatment of soft tissue sarcoma, wherein the method comprises determining if a patient will respond or is responding to treatment with a combined therapy comprising a PD-1/PD-L1 inhibitor and a tyrosine kinase by using any of the methods of the second aspect of the invention, or determining if a combined therapy comprising a PD-1/PD-L1 inhibitor and a tyrosine kinases is recommended by using any of the methods of third aspect of the invention.

In a preferred embodiment, the combination drug product comprises a PD-1/PD-L1 inhibitor and a tyrosine kinase inhibitor.

In a preferred embodiment the combination drug product is a combination of a PD-1/PD-L1 inhibitor and a tyrosine kinase inhibitor.

The soft tissue sarcomas, PD-1/PD-L1 inhibitors and tyrosine kinase inhibitors are the same as described for the first aspect. Thus, all the embodiments of the description mentioned in these regards for the first aspect apply herein.

A seventh aspect of the invention refers to a computer-implemented method, wherein a processing unit and a software are configured to receive expression values of the genes or combinations of genes of any of the methods of the first, second or third aspect of the invention, process the values received in order to find substantial variations or deviations with respect to a pre-established threshold value, and provide an output through a terminal display of the variation or deviation of the expression level.

Each embodiment disclosed herein is contemplated as being applicable to each of the other disclosed embodiments. Thus, all combinations of the various elements described herein are within the scope of the invention. It should also be understood that, unless clearly indicated otherwise, regarding the methods of the invention that include more than one step or act, the order of the steps or acts of the method is not necessarily limited to the order in which the steps or acts of the method are mentioned.

Unless otherwise stated, the terms used in the present application have the meanings indicated herein. Terms that have not been defined herein should be given the meanings that would be given to them by a person skilled in the art in the context of this disclosure.

The following terms are defined:
- The term "comprising" means including, but it is not limited to, whatever follows the word "comprising". When an invention is described as "comprising" certain elements, it means that the invention includes those elements, but it is not necessarily limited to those elements. Additional elements may be present as well. The word "comprise" includes the term "consists of'.
- The term "consisting of" means including, and it is limited to, whatever follows the phrase "consisting of'. When an invention is described as "consisting of" certain elements, it means that the invention includes only those elements and no others. This phase implies a more restrictive scope, where the invention is limited to the exact elements listed and does not encompass additional elements beyond them.
- The term "soft tissue sarcoma" refers to a type of cancer that originates in the soft tissues of the body, such as muscles, tendons, fat, nerves, blood vessels, and deep skin tissues. These tumours can occur in any part of the body but are most commonly found in the arms, legs, chest and abdomen.
- The term "a PD-1/PD-L1 inhibitor" refers to a type of cancer immunotherapy medication that works by blocking the interaction between programmed death receptor 1 (PD-1) on immune cells and its ligand (PD-L1) on cancer cells. By disrupting this interaction, PD-1/PD-L1 inhibitors help activate the body's immune system to attack and destroy cancer cells. They are used to treat various types of cancer, including melanoma, lung cancer, bladder cancer, and others.
- The term "tyrosine kinase inhibitor" refers to a type of medication that targets and blocks the activity of tyrosine kinases, which are enzymes involved in cell signalling pathways. Tyrosine kinases play a role in various cellular processes, including cell growth, proliferation, and survival. By inhibiting these enzymes, tyrosine kinase inhibitors interfere with the signals that promote cancer cell growth and division. Tyrosine kinase inhibitors are commonly used in the treatment of certain types of cancer, such as chronic myeloid leukaemia, non-small cell lung cancer, and gastrointestinal tumours.
- The term "enrichment" of a gene signature refers to a value that represents whether a predefined set of genes (gene signature) shows statistically significant overrepresentation within a particular biological context, such as a set of differentially expressed genes in a disease or under certain experimental conditions. This analysis helps understand the functional relevance of the gene signature within the context of interest.
- The term "responder" refers to an individual who demonstrates a positive response to a particular treatment or intervention. In the context of medical treatments, such as cancer therapies or medications, a responder patient experiences beneficial effects from the treatment, such as tumour shrinkage, symptom improvement, or disease stabilisation . Identifying responder patients helps healthcare providers tailor treatments more effectively and optimise patient care.
- The term "non-responder" refers to an individual who does not demonstrate a positive response to a particular treatment or intervention. In the context of medical treatments, such as cancer therapies or medications, a non-responder patient does not experience the desired beneficial effects from the treatment, such as tumour shrinkage, symptom improvement, or disease stabilisation . Identifying non-responder patients is important for healthcare providers to reassess treatment strategies and explore alternative options to optimise patient care.
- The term "biological sample" refers to any material derived from a living organism that is used for scientific analysis or experimentation. This can include tissues, cells, blood, saliva, urine, DNA, RNA, proteins, or any other biological material collected from a subj ect.
- The term "pre-established threshold value" refers to a predetermined level or limit set in advance, beyond which a certain action, condition, or response is triggered. It serves as a reference point for making decisions or taking actions based on specific criteria.
- The term "combination drug product" refers to a pharmaceutical formulation that contains two or more active ingredients combined in a single dosage form. These active ingredients may have different pharmacological actions or may work synergistically to enhance the therapeutic effects of the medication. Combination drug products are often used to treat complex conditions or to provide a more comprehensive treatment approach by targeting multiple aspects of a disease disorder.

### Description of the figures

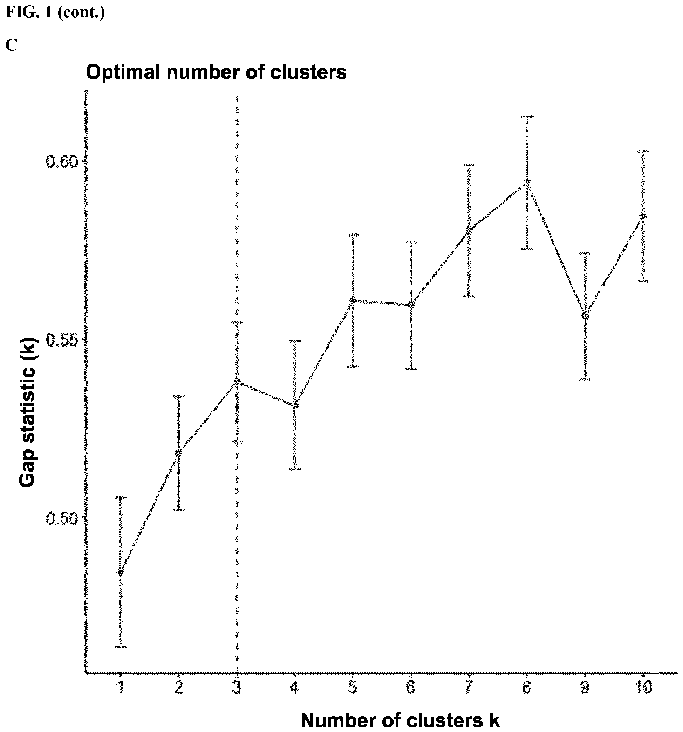

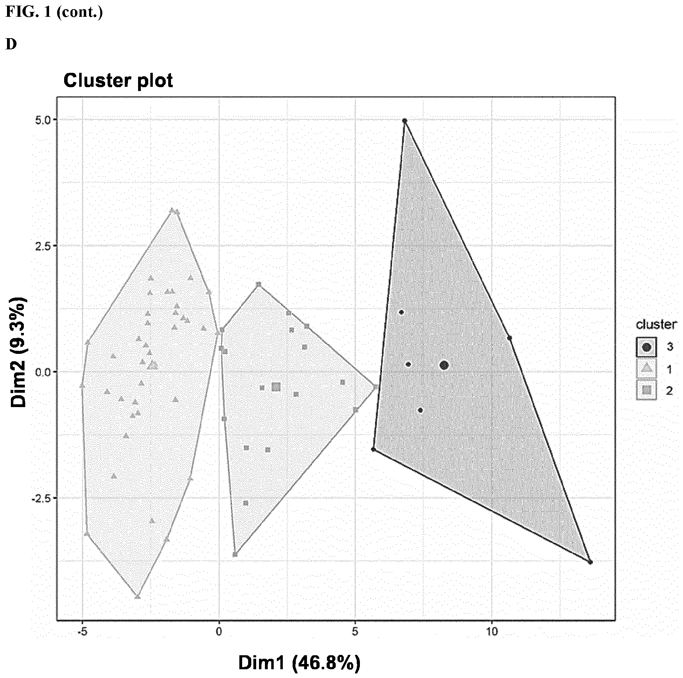

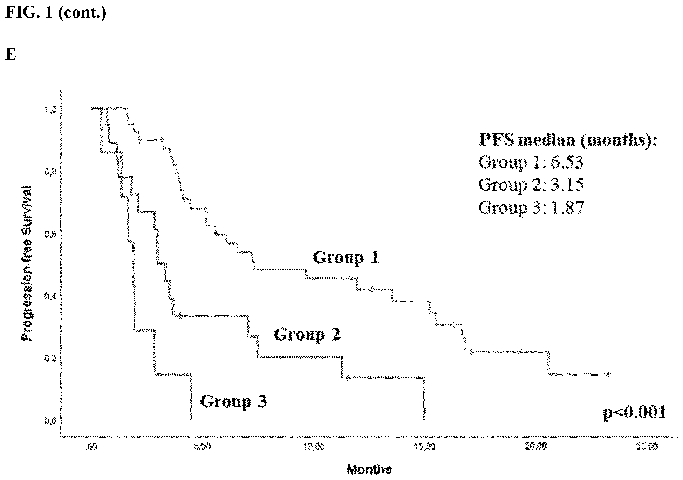

**Figure 1****.** Progression Free Survival (PFS) signature (a): list of 32 genes with differential expression in patients with PFS ≥ 6 months versus patients with PFS < 6 months. Heatmap for PFS signature (b): The selected genes were obtained from six Partial Least Squares (PLS) regression methods combined. Optimal clustering in PFS signature (c) for two not-parametric clustering procedures (K-mean clustering with gap statistics and K-mean clustering with silhouette method). Cluster plot (d) for the 64 patients' samples assumed to be pooled in 3 groups, according to the k-mean clustering method with Progression Free Survival (PFS) signature. Kaplan-Meier Survival curve (e) for the 64 patients' samples assumed to be pooled in 3 groups, according to the k-mean clustering method with Progression Free Survival (PFS) signature.
**Figure 2****.** GLM variable importance plot of progression-free survival gene signature composed by 32 genes.

### Detailed description of the invention

The present invention is illustrated by means of the Examples set below without the intention of limiting its scope of protection.

### Example 1. Material and Methods

### Example 1.1. Patients and samples

Tumour biopsies were obtained from 64 patients with different subtypes of soft tissue sarcoma (STS), within the 28 days before treatment initiation with a combination of nivolumab and sunitinib.

### Example 1.2. Gene expression assay (HTG)

RNA was extracted from FFPE tumour samples (5 slides of 5 µm) with the QIAamp FFPE Tissue Kit (Qiagen,Valencia, CA), according to manufacturer's instructions.

Gene expression analysis of 2549 human RNA transcripts related to tumour biology was performed with HTG EdgeSeq technology (HTG Molecular Diagnostics, Tucson, AZ, USA), using the EdgeSeq Oncology Biomarker Panel (OBP) (https://www.htgmolecular.com/assays/obp, accessed on 12 September 2020) and following the specific instructions and recommendations for sequencing with the Illumina technology. Demultiplexed FASTQ files were aligned with the probe list using the HTG EdgeSeq host software. The results were parsed, and the output was obtained as a read count matrix.

### Example 1.3. Bioinformatics analysis

The data analysis was conducted in R, utilising Bioconductor and plotting packages.

### Differential Expression Analysis

The raw gene counts were normalised, based on batch and patient effect, and analysed by DESeq2 to identify differentially expressed genes in STS patients with progression-free survival (PFS) ≥ 6 months versus PFS<6 months. Only genes with a fold change ≤2 or ≥2 (log2foldchange ≥1 or ≤-1) and a p-adjusted value (False Discovery Rate, FDR) lower than 5% were considered for the signature refinement.

### Signature Refinement using Partial Least Square (PLS) Methods

Genes with differential expression previously selected were refined with the plsVarSel R package using six variable selection methods that were run 100 times each: BVE_PLS: A backward variable elimination procedure for elimination of non-informative variables; GA_PLS: A subset search algorithm inspired by biological evolution theory and natural selection; IPW_PLS: An iterative procedure for variable elimination; REP_PLS: A regularised variable elimination procedure for parsimonious variable selection, where also a stepwise elimination is carried out; MCUVE_PLS: Artificial noise variables are added to the predictor set before the PLSR model is fitted. All the original variables having lower importance than the artificial noise variables are eliminated before the procedure is repeated until a stop criterion is reached; SPA_PLS provides the influence of each variable without considering the influence of the rest of the variables through sub-sampling of samples and variables. The genes that appeared in at least 100% of one repetition were combined to form the final PFS signature.

### Clustering Analysis

The normalised data from the final PFS signature was plotted as heatmap and subjected to unsupervised k-mean clustering to identify distinct risk groups. The optimal number of clusters was determined using Gap statistics and Average Silhouette methods.

### Survival Analysis

Kaplan-Meier curves were plotted to assess the association between the groups generated by k-mean gene signature and PFS clinical endpoint.

### PFS Predictive Models

To predict patients' survivability based on the PFS gene signature, a GLM was fitted using the R package "glm". The panel of influential genes was sorted based on the Akaike Information Criterion (AIC) parameter, employing Leave-One-Out cross-validation for model evaluation. Additionally, each gene's importance coefficient was sorted to identify key contributors to the predictive model.

### Example 2. Results

### Example 2.1. Patients' demographics

A total of 64 STS patients treated within the multicenter, single-arm, phase Ib/II clinical trial, testing the combination of an anti-angiogenic agent, sunitinib, with an anti-PD-1 immune checkpoint inhibitor, nivolumab were used for this IMMUNOSARC correlative study. The median age was 40 (20-78) years with 59% (n=38) and 41% (n=26) being male and female, respectively. A total of 94% (n=60) of the patients had metastatic disease at the beginning of treatment (baseline), while 6% (n=4) had unresectable locally advanced disease. The ECOG status at baseline was 0 and 1 in 47% (n=30) and 53% (n=34) of the cases, respectively. The most frequent subtypes, according to central pathology review were clear cell sarcoma (17%; n=11), synovial sarcoma (17%; n=11) and undifferentiated pleomorphic sarcoma (UPS; 16 %; n=10). Other subtypes included were alveolar soft-part sarcoma (ASPS; 11%; n=7), angiosarcoma (9.3%; n=6), epithelioid sarcoma (11%; n=7), solitary fibrous tumour (SFT; 11%; n=7), extraskeletal myxoid chondrosarcoma (EMC; 6%; n=4) and epithelioid hemangioendothelioma (2%; n=1) **(Table 1).**

**Table 1. Number of patients studied per sarcoma subtype.**

| **Sarcoma Subtype** | **N. of patients** |
|---|---|
| Alveolar soft part sarcoma | 7 |
| Angiosarcoma | 6 |
| Clear cell sarcoma | 11 |
| Epithelioid sarcoma | 7 |
| Extraskeletal myxoid chondrosarcoma | 4 |
| Solitary fibrous tumour | 7 |
| Synovial sarcoma | 11 |
| Undifferentiated pleomorphic sarcoma | 10 |
| Epithelioid hemangioendothelioma | 1 |
| Total | 64 |

### Example 2.2. Predictive gene-based signature

The gene expression profile of FFPE tumour samples from 64 different subtypes of soft tissue sarcoma patients were analysed before treatment with nivolumab plus sunitinib. A total of 118 genes were significantly and differential expressed at least two folds in patients with a progression free survival (PFS) ≥ 6 months (n=38) versus patients with PFS < 6 months (n=26). Among them, 51 genes were overexpressed in patients with worse PFS, and 67 genes were overexpressed in patients with better PFS. The pool of genes differentially expressed was further trimmed with the PLS method in a series of 100 repetitions, to build a PFS gene-based signature which finally contained 32 genes *(ANLN, AURKB, C5AR1, CCNA2, CCNB1, CDC20, CDC7, CENPF, DLGAP5, DNMT1, E2F1, E2F3, ECT2, FAM105A, GPR126, HIST1H3H, LRRK2, MAOB, MELK, MK167, NTHL1, NUF2, NUSAP1, PPAP2B, RRM2, RUNX2, SKP2, STMN1, TFDP1, TOP2A, TPX2* and *TYMS)* **(****Figure 1a****).** Functional enrichment analysis showed that the genes included in the PFS gene-based signature were mostly associated with processes related with cell cycle regulation, according to the *BIOCARTA* database, and with immunomodulation mechanisms, in accordance with *AZIMUTH* database, supporting the potential involvement of these genes in the response to immunotherapy-based regimens **(****Figure 1a****).**

A row-scaled heatmap of normalised counts from the 32 genes of the PFS gene signature clustered our study population in 3 separated risk groups **(****Figure 1b****).** Gene expression heatmap characterises group 1 by lower gene expression intensities. Since DEseq2 reference level was set PFS < 6 months, the reduction of expression in group 1 is in accordance with most of the log fold changes being negative (27 out of 32 genes). K-mean optimal clustering methods, according to unsupervised Gap-statistics, confirmed the number of three risk groups **(****Figure 1c****).** Kaplan Meier analysis rendered statistically significant differences among those three groups (p-value < 0.001), being group 1 the one that exhibited the highest median PFS (6.53 months (95 % CI 0.8-13.8), n=39) versus group 2 (3.15 months (95% CI 2.3-3.7), n=18) and group 3 (1.87 months (95% CI 1.3-2.5), n=7) (p<0.001).

The marginal and accumulative importance of each gene on the PFS gene signature was also represented in GLM variable importance plots, showing than only 9 genes *(DLGAP5, MELK, C5AR1, TPX2, NUF2, CDC20, DNMT1, CDC7* and *E2F1)* explained more than 75% of the PFS variance **(****Figure 2****).**

## Claims

1. An *in vitro* method for the identification of biomarker signatures suitable for predicting the response of a patient suffering from soft tissue sarcoma to treatment with a combined therapy comprising a PD-1/PD-L1 inhibitor and a tyrosine kinase inhibitor, or for classifying a patient suffering from soft tissue sarcoma into responder or non-responder patients to a treatment with a combined therapy comprising a PD-1/PD-L1 inhibitor and a tyrosine kinase inhibitor, or for monitoring patients suffering from soft tissue sarcoma to assess whether they are responding to a treatment with a combined therapy comprising a PD-1/PD-L1 inhibitor and a tyrosine kinase inhibitor, or for deciding or recommending whether to treat a patient suffering from soft tissue sarcoma treatment with a combined therapy comprising a PD-1/PD-L1 inhibitor and a tyrosine kinase inhibitor which comprises:
a) assessing the level of expression of at least two genes selected from the list consisting of: DLGAP5, ANLN, AURKB, C5AR1, CCNA2, CCNB1, CDC20, CDC7, CENPF, DNMT1, E2F1, E2F3, ECT2, FAM105A, GPR126, HIST1H3H, LRRK2, MAOB, MELK, MKI67, NTHL1, NUF2, NUSAP1, PPAP2B, RRM2, RUNX2, SKP2, STMN1, TFDP1, TOP2A, TPX2 and/or TYMS, or the enrichment of the signature comprising said genes, in a biological sample obtained from a patient suffering from soft tissue sarcoma who is a responder patient to treatment with a combined therapy comprising a PD-1/PD-L1 inhibitor and a tyrosine kinase inhibitor,
b) assessing the level of expression of the at least two genes selected from the list consisting of: DLGAP5, ANLN, AURKB, C5AR1, CCNA2, CCNB1, CDC20, CDC7, CENPF, DNMT1, E2F1, E2F3, ECT2, FAM105A, GPR126, HIST1H3H, LRRK2, MAOB, MELK, MKI67, NTHL1, NUF2, NUSAP1, PPAP2B, RRM2, RUNX2, SKP2, STMN1, TFDP1, TOP2A, TPX2 and/or TYMS, or the enrichment of the signature comprising said genes, in a biological sample obtained from a patient suffering from soft tissue sarcoma who is a non-responder patient to treatment with a combined therapy comprising a PD-1/PD-L1 inhibitor and a tyrosine kinase inhibitor,
c) wherein the identification of a deviation in the expression level of the at least two genes selected from selected from the list consisting of: DLGAP5, ANLN, AURKB, C5AR1, CCNA2, CCNB1, CDC20, CDC7, CENPF, DNMT1, E2F1, E2F3, ECT2, FAM105A, GPR126, HIST1H3H, LRRK2, MAOB, MELK, MKI67, NTHL1, NUF2, NUSAP1, PPAP2B, RRM2, RUNX2, SKP2, STMN1, TFDP1, TOP2A, TPX2 and/or TYMS, or the enrichment of the signature comprising said genes, across responder and non-responder patients, is an indication that the signature is suitable for predicting the response of a patient suffering from soft tissue sarcoma to treatment with a combined therapy comprising a PD-1/PD-L1 inhibitor and a tyrosine kinase inhibitor, or for classifying a patient suffering from soft tissue sarcoma into responder or non-responder patients to a treatment with a combined therapy comprising a PD-1/PD-L1 inhibitor and a tyrosine kinase inhibitor, or for monitoring patients suffering from soft tissue sarcoma to assess whether they are responding to a treatment with a combined therapy comprising a PD-1/PD-L1 inhibitor and a tyrosine kinase inhibitor, or for deciding or recommending whether to treat a patient suffering from soft tissue sarcoma treatment with a combined therapy comprising a PD-1/PD-L1 inhibitor and a tyrosine kinase inhibitor.

2. The *in vitro* method, according to claim 1, which comprises:
a) assessing the level of expression of at least two genes selected from the list consisting of: DLGAP5, CDC7, CDC20, C5AR1, DNMT1, E2F1, MELK, NUF2 and/or TPX2, or the enrichment of the signature comprising said genes, in a biological sample obtained from a patient suffering from soft tissue sarcoma who is a responder patient to treatment with a combined therapy comprising a PD-1/PD-L1 inhibitor and a tyrosine kinase inhibitor,
b) assessing the level of expression of the at least two genes selected from the list consisting of: DLGAP5, CDC7, CDC20, C5AR1, DNMT1, E2F1, MELK, NUF2 and/or TPX2, or the enrichment of the signature comprising said genes, in a biological sample obtained from a patient suffering from soft tissue sarcoma who is a non-responder patient to treatment with a combined therapy comprising a PD-1/PD-L1 inhibitor and a tyrosine kinase inhibitor,
c) wherein the identification of a deviation in the expression level of the at least two genes selected from selected from the list consisting of: DLGAP5, CDC7, CDC20, C5AR1, DNMT1, E2F1, MELK, NUF2 and/or TPX2, or the enrichment of the signature comprising said genes, across responder and non-responder patients, is an indication that the signature is suitable for predicting the response of a patient suffering from soft tissue sarcoma to treatment with a combined therapy comprising a PD-1/PD-L1 inhibitor and a tyrosine kinase inhibitor, or for classifying a patient suffering from soft tissue sarcoma into responder or non-responder patients to a treatment with a combined therapy comprising a PD-1/PD-L1 inhibitor and a tyrosine kinase inhibitor, or for monitoring patients suffering from soft tissue sarcoma to assess whether they are responding to a treatment with a combined therapy comprising a PD-1/PD-L1 inhibitor and a tyrosine kinase inhibitor, or for deciding or recommending whether to treat a patient suffering from soft tissue sarcoma treatment with a combined therapy comprising a PD-1/PD-L1 inhibitor and a tyrosine kinase inhibitor.

3. An *in vitro* method for predicting the response of a patient suffering from soft tissue sarcoma to treatment with a combined therapy comprising a PD-1/PD-L1 inhibitor and a tyrosine kinase inhibitor, or for classifying a patient suffering from soft tissue sarcoma into responder or non-responder patient to a treatment with a combined therapy comprising a PD-1/PD-L1 inhibitor and a tyrosine kinase inhibitor, or for monitoring patients suffering from soft tissue sarcoma to assess whether they are responding to treatment with a combined therapy comprising a PD-1/PD-L1 inhibitor and a tyrosine kinase inhibitor, which comprises:
a) assessing the level of expression of a combination of at least two genes selected from the list consisting of: DLGAP5, CDC7, CDC20, C5AR1, DNMT1, E2F1, MELK, NUF2 and/or TPX2 in a biological sample obtained from the patient,
b) wherein the identification of a decreased expression level of the genes DLGAP5, CDC7, CDC20, DNMT1, E2F1, MELK, NUF2 and/or TPX2 and an increased expression level of gene C5AR1, as compared with a pre-established threshold value, is an indication that the patient will respond to treatment with a combined therapy comprising a PD-1/PD-L1 inhibitor and a tyrosine kinase inhibitor, or that the patient is responding to treatment with a combined therapy comprising a PD-1/PD-L1 inhibitor and a tyrosine kinase inhibitor,
c) wherein the identification of an increased expression level of the genes DLGAP5, CDC7, CDC20, DNMT1, E2F1, MELK, NUF2 and/or TPX2 and a decreased expression level of gene C5AR1, as compared with a pre-established threshold value, is an indication that the patient will not respond to treatment with a combined therapy comprising a PD-1/PD-L1 inhibitor and a tyrosine kinase inhibitor, or that the patient is not responding to treatment with a combined therapy comprising a PD-1/PD-L1 inhibitor and a tyrosine kinase inhibitor,
d) wherein the pre-established threshold value is determined in patients that do not respond to treatment with a combined therapy comprising a PD-1/PD-L1 inhibitor and a tyrosine kinase inhibitor, or by comparing patients that respond to treatment with a combined therapy comprising a PD-1/PD-L1 inhibitor and a tyrosine kinase inhibitor with patients that do not respond to treatment with a combined therapy comprising a PD-1/PD-L1 inhibitor and a tyrosine kinase inhibitor.

4. An *in vitro* method for deciding or recommending whether to treat a patient suffering from soft tissue sarcoma treatment with a combined therapy comprising a PD-1/PD-L1 inhibitor and a tyrosine kinase inhibitor, which comprises:
a) assessing the level of expression of a combination of at least two genes selected from the list consisting of: DLGAP5, CDC7, CDC20, C5AR1, DNMT1, E2F1, MELK, NUF2 and/or TPX2 in a biological sample obtained from the patient,
b) wherein the identification of a decreased expression level of the genes DLGAP5, CDC7, CDC20, DNMT1, E2F1, MELK, NUF2 and/or TPX2 and an increased expression level of gene C5AR1, as compared with a pre-established threshold value, is an indication that treatment with a combined therapy comprising a PD-1/PD-L1 inhibitor and a tyrosine kinase inhibitor is recommended,
c) wherein the identification of an increased expression level of the genes DLGAP5, CDC7, CDC20, DNMT1, E2F1, MELK, NUF2 and/or TPX2 and a decreased expression level of gene C5AR1, as compared with a pre-established threshold value, is an indication that treatment with a combined therapy comprising a PD-1/PD-L1 inhibitor and a tyrosine kinase inhibitor is not recommended,
d) wherein the pre-established threshold value is determined in patients that do not respond to treatment with a combined therapy comprising a PD-1/PD-L1 inhibitor and a tyrosine kinase inhibitor, or by comparing patients that respond to treatment with a combined therapy comprising a PD-1/PD-L1 inhibitor and a tyrosine kinase inhibitor with patients that do not respond to treatment with a combined therapy comprising a PD-1/PD-L1 inhibitor and a tyrosine kinase inhibitor.

5. *In vitro* use of the level of expression of a combination of at least two genes selected from the list consisting of DLGAP5, CDC7, CDC20, C5AR1, DNMT1, E2F1, MELK, NUF2 and/or TPX2 for predicting the response of a patient suffering from soft tissue sarcoma to treatment with a combined therapy comprising a PD-1/PD-L1 inhibitor and a tyrosine kinase inhibitor, for classifying a patient suffering from soft tissue sarcoma into responder or non-responder patient to treatment with a combined therapy comprising a PD-1/PD-L1 inhibitor and a tyrosine kinase inhibitor, for monitoring patients suffering from soft tissue sarcoma to assess whether they are responding to treatment with a combined therapy comprising a PD-1/PD-L1 inhibitor and a tyrosine kinase inhibitor, or for the identification of biomarker signatures suitable for predicting the response of a patient suffering from soft tissue sarcoma to treatment with a combined therapy comprising a PD-1/PD-L1 inhibitor and a tyrosine kinase inhibitor
or *in vitro* use of a kit comprising reagents for the assessment of the level of expression of a combination of at least two genes selected from the list consisting of DLGAP5, CDC7, CDC20, C5AR1, DNMT1, E2F1, MELK, NUF2 and/or TPX2 for predicting the response of a patient suffering from soft tissue sarcoma to treatment with a combined therapy comprising a PD-1/PD-L1 inhibitor and a tyrosine kinase inhibitor, for classifying a patient suffering from soft tissue sarcoma into responder or non-responder patient to treatment with a combined therapy comprising a PD-1/PD-L1 inhibitor and a tyrosine kinase inhibitor, for monitoring patients suffering from soft tissue sarcoma to assess whether they are responding to treatment with a combined therapy comprising a PD-1/PD-L1 inhibitor and a tyrosine kinase inhibitor, or for the identification of biomarker signatures suitable for predicting the response of a patient suffering from soft tissue sarcoma to treatment with a combined therapy comprising a PD-1/PD-L1 inhibitor and a tyrosine kinase inhibitor.

6. The *in vitro* method, according to any of the claims 3 to 4, or the *in vitro* use, according to claim 5, which comprises:
a) assessing the level of expression of the combination of genes selected consisting of: DLGAP5, CDC7, CDC20, C5AR1, DNMT1, E2F1, MELK, NUF2 and TPX2 in a biological sample obtained from the patient,
b) wherein the identification of a decreased expression level of the genes DLGAP5, CDC7, CDC20, DNMT1, E2F1, MELK, NUF2 and TPX2, and an increased expression level of gene C5AR1, as compared with a pre-established threshold value, is an indication that the patient will respond to treatment with a combined therapy comprising a PD-1/PD-L1 inhibitor and a tyrosine kinase inhibitor, or that the patient is responding to treatment with a combined therapy comprising a PD-1/PD-L1 inhibitor and a tyrosine kinase inhibitor, or that treatment with a combined therapy comprising a PD-1/PD-L1 inhibitor and a tyrosine kinase inhibitor is recommended,
c) wherein the identification of an increased expression level of the genes DLGAP5, CDC7, CDC20, DNMT1, E2F1, MELK, NUF2 and TPX2, and a decreased expression level of gene C5AR1, as compared with a pre-established threshold value, is an indication that the patient will not respond to treatment with a combined therapy comprising a PD-1/PD-L1 inhibitor and a tyrosine kinase inhibitor, or that the patient is not responding to treatment with a combined therapy comprising a PD-1/PD-L1 inhibitor and a tyrosine kinase inhibitor, or that treatment with a combined therapy comprising a PD-1/PD-L1 inhibitor and a tyrosine kinase inhibitor is not recommended,
d) wherein the pre-established threshold value is determined in patients that do not respond to treatment with a combined therapy comprising a PD-1/PD-L1 inhibitor and a tyrosine kinase inhibitor, or by comparing patients that respond to treatment with a combined therapy comprising a PD-1/PD-L1 inhibitor and a tyrosine kinase inhibitor with patients that do not respond to treatment with a combined therapy comprising a PD-1/PD-L1 inhibitor and a tyrosine kinase inhibitor.

7. The *in vitro* method, according to any of the claims 1 to 4 or 6, or *in vitro* use, according to any of the claims 5 or 6, wherein the biological sample is a soft tissue sarcoma tissue biopsy.

8. The *in vitro* method, according to any of the claims 1 to 4, 6 or 7, or *in vitro* use, according to any of the claims 5 to 7, wherein the PD-1/PD-L1 inhibitor is selected from:
a) a PD-1 inhibitor selected from: nivolumab, pembrolizumab, cemiplimab, dostarlimab, retifanlimab and/or toripalimab, and/or
b) a PD-L1 inhibitor selected from: atezolizumab, avelumab and/or durvalumab,

9. The *in vitro* method, according to any of the claims 1 to 4 and 6 to 8, or *in vitro* use, according to any of the claims 5 to 8, wherein the tyrosine kinase inhibitor is selected from: sunitinib, pazopanib, cediranib, axitinib, anlotinib, apatinib, sorafenib and/or regorafenib

10. The *in vitro* method, according to any of the claims 1 to 4, 6 to 9, or *in vitro* use, according to any of the claims 5 to 9, wherein the PD-1/PD-L1 inhibitor is nivolumab and/or wherein the tyrosine kinase inhibitor is sunitinib.

11. The *in vitro* method, according to any of the claims 3 or 4, wherein the combination of genes consists of DLGAP5, CDC7, CDC20, C5AR1, DNMT1, E2F1, MELK, NUF2 and TPX2, wherein the biological sample is a soft tissue sarcoma tissue biopsy, wherein the PD-1/PD-L1 inhibitor is nivolumab and wherein the tyrosine kinase inhibitor is sunitinib.

12. A combination drug product for use in a method of treatment of soft tissue sarcoma, wherein the method comprises determining if a patient will respond or is responding to treatment with a combined therapy comprising a PD-1/PD-L1 inhibitor and a tyrosine kinase by using any of the methods of claims 3 and 6 to 11, or determining if a combined therapy comprising a PD-1/PD-L1 inhibitor and a tyrosine kinases is recommended by using any of the methods of claims 4 and 6 to 11.

13. The combination drug product, according to claim 12, comprising a PD-1/PD-L1 inhibitor and a tyrosine kinase inhibitor for use in the treatment of soft tissue sarcoma, wherein the PD-1/PD-L1 inhibitor is selected from:
a) a PD-1 inhibitor selected from: nivolumab, pembrolizumab, cemiplimab, dostarlimab, retifanlimab and/or toripalimab, and/or
b) a PD-L1 inhibitor selected from: atezolizumab, avelumab and/or durvalumab.

14. The combination drug product, according to any of the claims 12 or 13, wherein the tyrosine kinase inhibitor is selected from: sunitinib, pazopanib, cediranib, axitinib, anlotinib, apatinib, sorafenib and/or regorafenib.

15. The combination drug product, according to any of the claims 12 to 14, wherein the PD-1/PD-L1 inhibitor is nivolumab and/or wherein the tyrosine kinase inhibitor is sunitinib.
